# EUROPEAN PATENT APPLICATION

(11) **EP 4 253 518 A1**
(43) Date of publication of application: **04.10.2023**
(21) Application number: 23164880.9
(22) Date of filing: 29.03.2023
(51) Int. Cl.: C12M 1/12

(54) **CELL CULTURE ARRANGEMENT, DEVICE AND METHOD OF USE**

(30) Priority: 31.03.2022 US 202263326085 P; 22.03.2023 US 202318124826
(71) Applicant: Pall Corporation, Port Washington, NY 11050 (US); Pall Life Sciences Belgium BV, 3320 Hoegaarden (BE); Pall Artelis BV, 3320 Hoegaarden (BE)
(72) Inventor: JACOBS, Nick J., 3320 Hoegaarden (BE); DOUCET, Frederik, 3320 Hoegaarden (BE); COLLARD, François, 3320 Hoegaarden (BE)
(74) Representative: Hoeger, Stellrecht & Partner Patentanwälte mbB

(57) **Abstract**

A cell culture device comprising a fixed bed arranged in a housing, the fixed bed comprising a continuous pleated porous medium comprising a top end, a bottom end, a front side, a rear side, a right side, and a left side, and a plurality of pleats folded on the right side and the left side, and having vertical fluid flow channels along the longitudinal axis between adjacent pleats, is disclosed.

## Description

### CROSS-REFERENCE TO RELATED APPLICATIONS

This patent application claims the benefit of U.S. Provisional Patent Application No. 63/326,085, filed March 31, 2022 which is incorporated by reference.

### BACKGROUND OF THE INVENTION

Cells can be cultured in fixed beds, wherein the cells adhere to porous carriers. However, there is a need for improved fixed beds.

The present invention provides for ameliorating at least some of the disadvantages of the prior art. These and other advantages of the present invention will be apparent from the description as set forth below.

### BRIEF SUMMARY OF THE INVENTION

An aspect of the invention provides a cell culture arrangement comprising (a) a housing having a first end and a second end and a longitudinal axis; and; (b) at least one fixed bed arranged in the housing, the at least one fixed bed comprising a continuous pleated porous medium comprising a top end, a bottom end, a front side, a rear side, a right side, and a left side, and a plurality of pleats folded on the right side and the left side, and having vertical fluid flow channels along the longitudinal axis between adjacent pleats.

In some aspects of the cell culture arrangement, the at least one fixed bed includes at least one removable sampling element comprising a porous medium arranged in the continuous pleated porous medium.

In another aspect, a cell culture device comprises (a) an aspect of the cell culture arrangement; (b) a pump body arranged at the second end of the housing, the pump body including a central cavity; (c) an impeller arranged in the central cavity of the pump body; and; (d) a cell culture vessel having a central chamber, wherein the cell culture arrangement is arranged in the central chamber.

In another aspect, a cell culture device comprises (a) an aspect of the cell culture arrangement; (b) a pump assembly arranged at the second end of the housing, the pump assembly comprising a pump outer body, and a pump body connected to the pump outer body, the pump body including a central cavity; (c) an impeller arranged in the central cavity of the pump body; and; (d) a cell culture vessel having an open first vessel end and a closed second vessel end, and a vessel side wall connected to the open first vessel end and the closed second vessel end, the cell culture vessel having a central chamber, wherein the cell culture arrangement and pump assembly are arranged in the central chamber.

A method of culturing cell according to an aspect of the invention comprises placing cell culture media and cells in an aspect of the cell culture device; and, operating the magnetic impeller. In some aspects, the method further comprises harvesting the cultured cells.

### BRIEF DESCRIPTION OF THE SEVERAL VIEWS OF THE DRAWING(S)

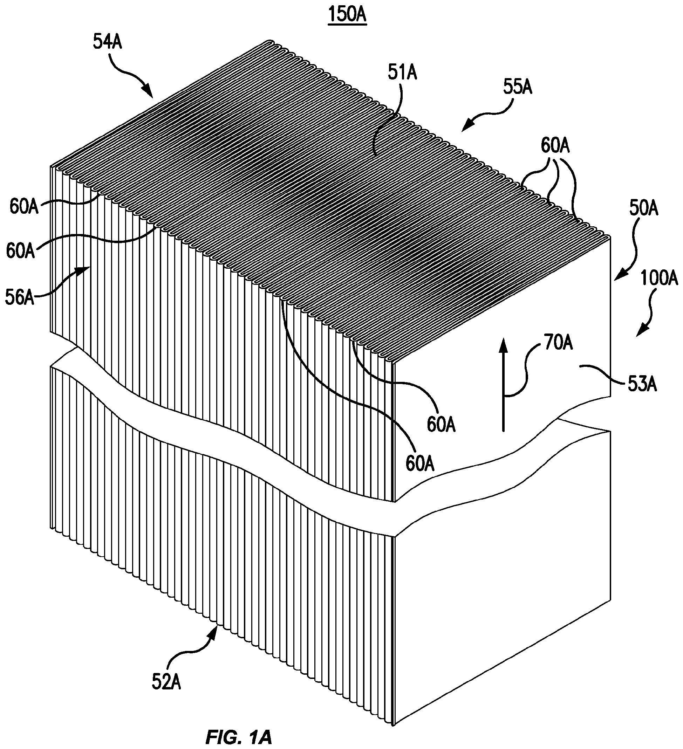

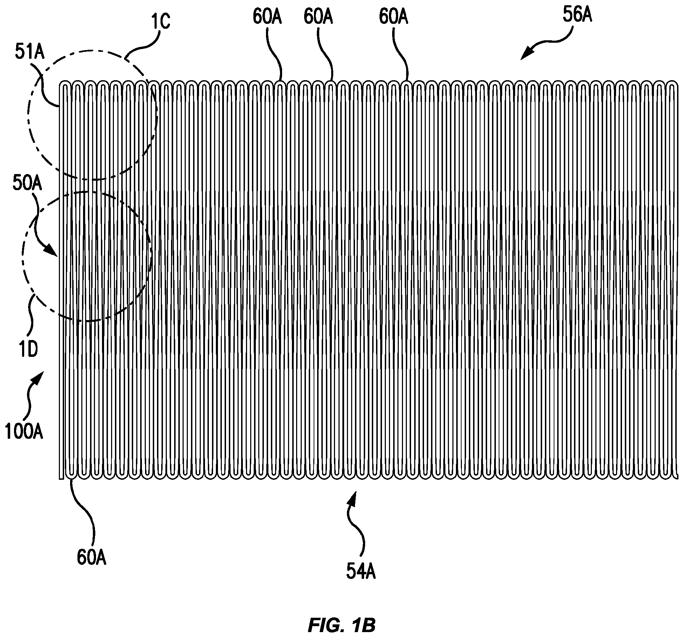

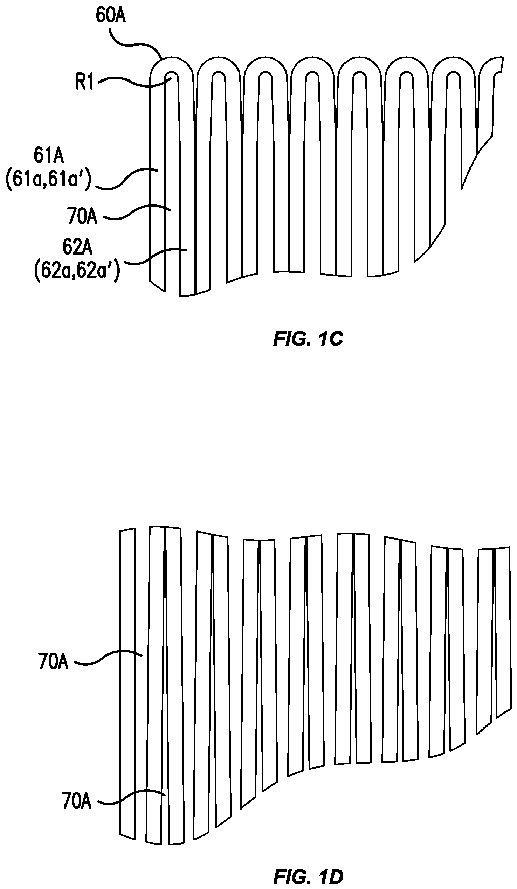

Figures 1A-1D are drawings showing representative views of a "dry" fixed bed comprising a pleated porous cell culture medium according to an aspect of the invention. Figure 1A shows an isometric view (wherein the bed has a rectangular configuration), Figure 1B shows a top view, Figures 1C and 1D show, respectively, enlarged views of portions "1C" and "1D" in Figure 1B.

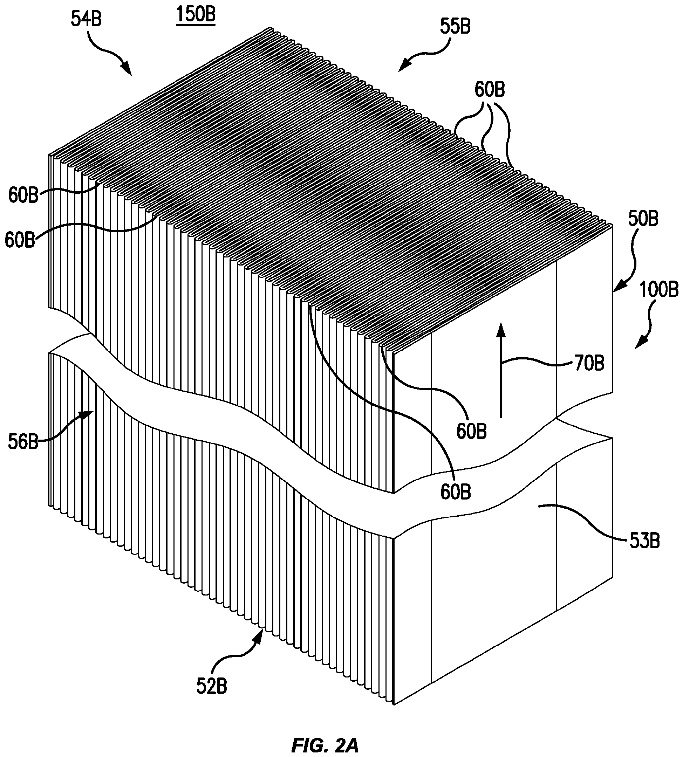

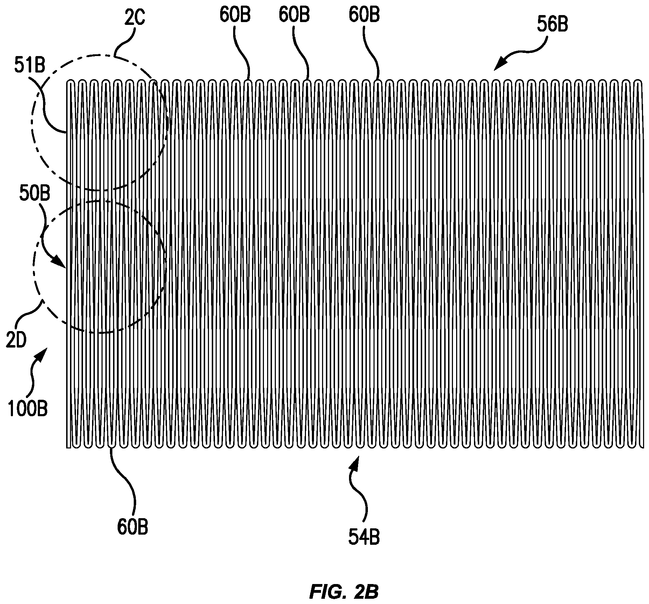

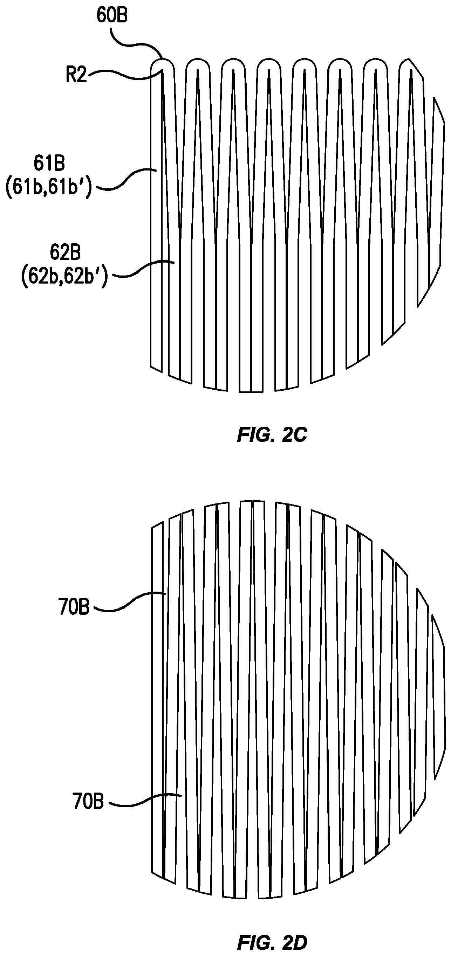

Figures 2A-2D are drawings showing representative views of a "dry" fixed bed comprising a pleated porous cell culture medium according to another aspect of the invention. Figure 2A shows an isometric view (wherein the bed has a rectangular configuration), Figure 2B shows a top view, Figures 2C and 2D show, respectively, enlarged views of portions "2C" and "2D" in Figure 2B.

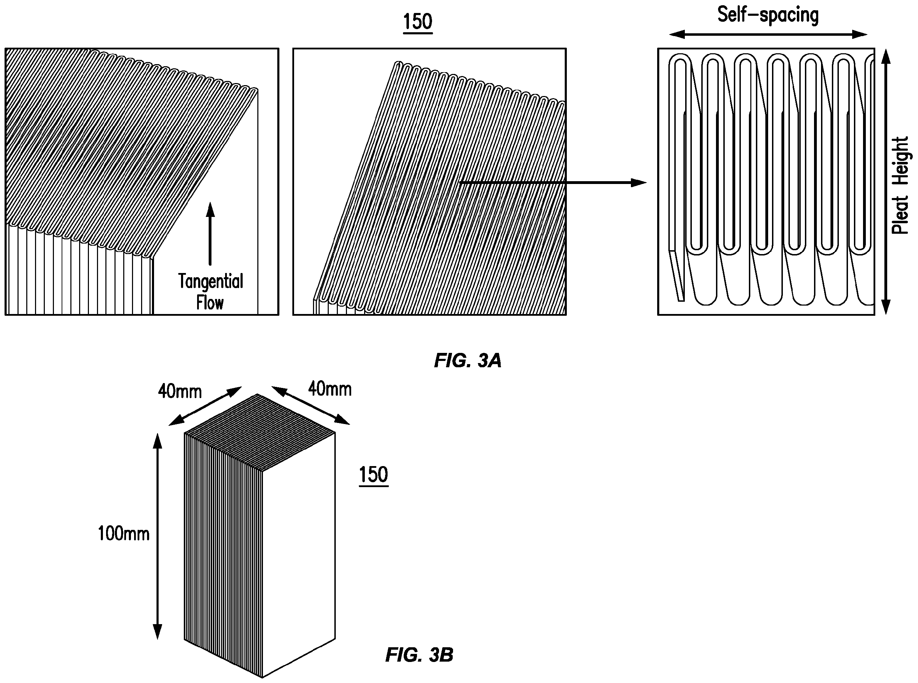

Figure 3A is a drawing showing tangential flow through the fixed bed and "self-spacing" resulting from the tangential fluid flow of cell culture liquid (liquid culture media) between each pair of legs of each pleat, also showing pleat height; Figure 3B is a drawing showing an isometric view of a pleated fixed bed having a rectangular configuration according to another aspect of the invention, also showing an exemplary fixed bed height.

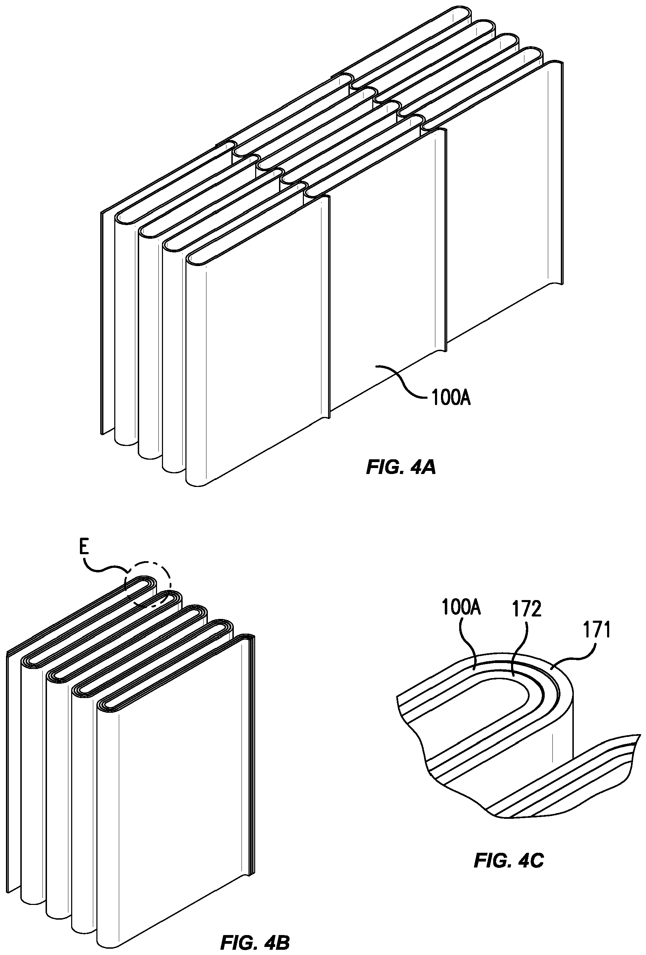

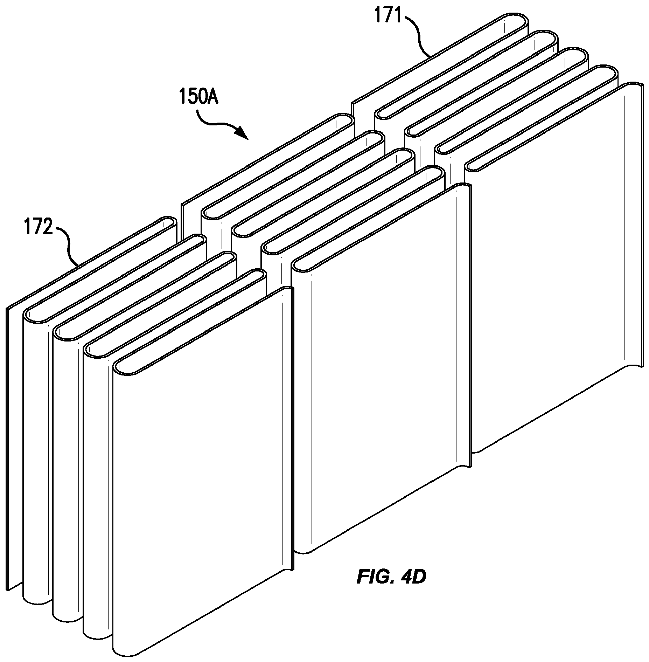

Figures 4A-4D are drawings showing the use of scrap material as "sacrificial layers" to produce the fixed bed shown in Figure 1A. Figure 4A shows insertion of scrap material on both sides of the porous cell culture medium; Figure 4B shows the pleated porous cell culture medium with pleated scrap material on either side of the culture medium; Figure 4C shows an enlarged view of portion "E" shown in Figure 4B; and Figure 4D shows the fixed bed after removal of the scrap material.

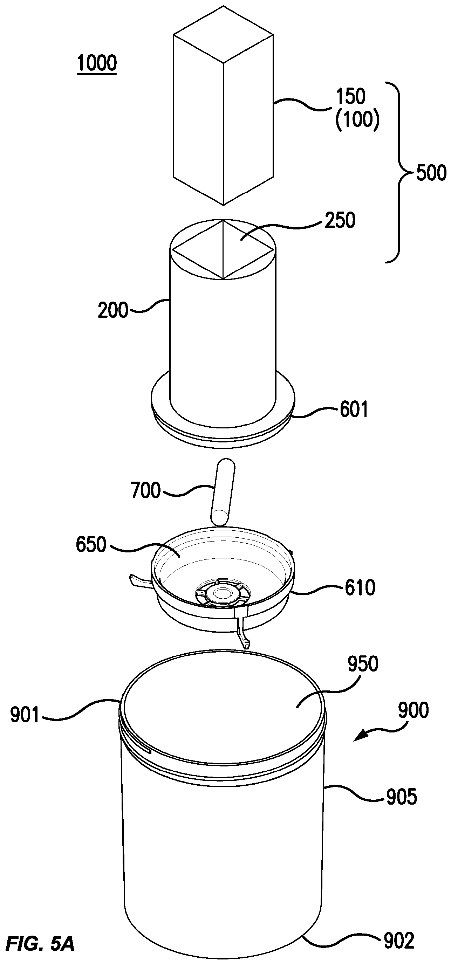

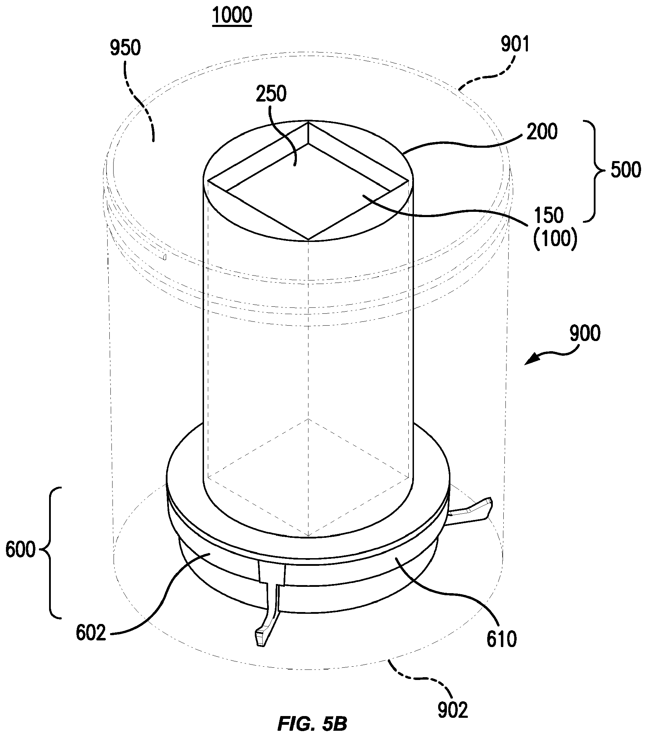

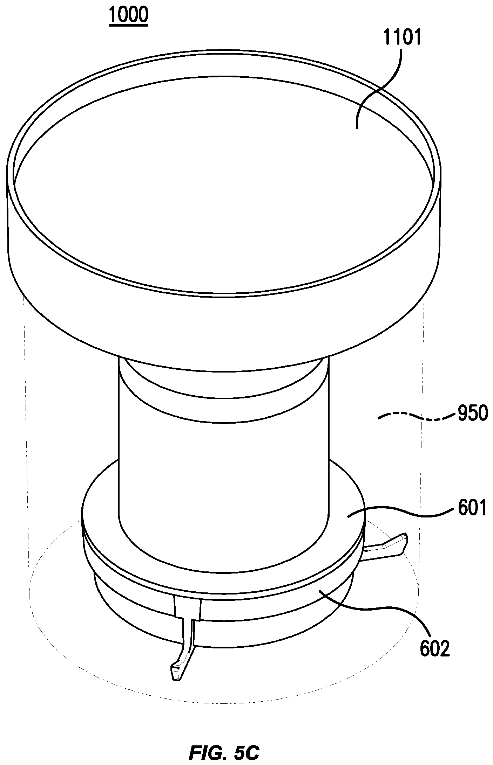

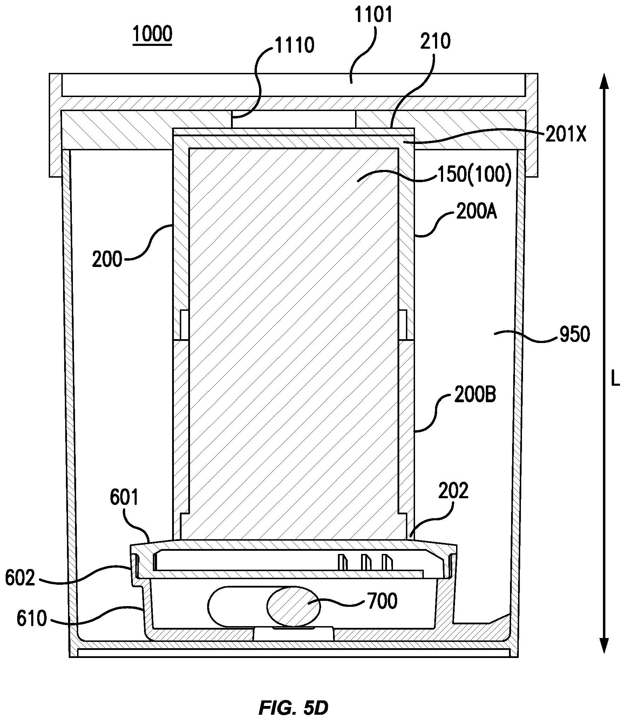

Figures 5A-5D are drawings showing a cell culture arrangement including the fixed bed comprising the pleated porous cell culture medium in a bed housing according to another aspect of the invention, wherein the cell culture arrangement is arranged in a vessel providing a cell culture device according to another aspect of the invention. Figure 5A shows an exploded view of the cell arrangement and the cell culture device; Figure 5B shows an isometric assembled view of the cell culture device; Figure 5C shows a perspective assembled view of the cell culture device for shipping/storage, also showing a shipping cover, and Figure 5D shows a cross-sectional assembled view for shipping/storage, also showing the shipping cover and a fixed bed retainer and a stabilizing element interposed between the shipping cover and the bed retainer, and an impeller arranged in the pump body below the bed housing.

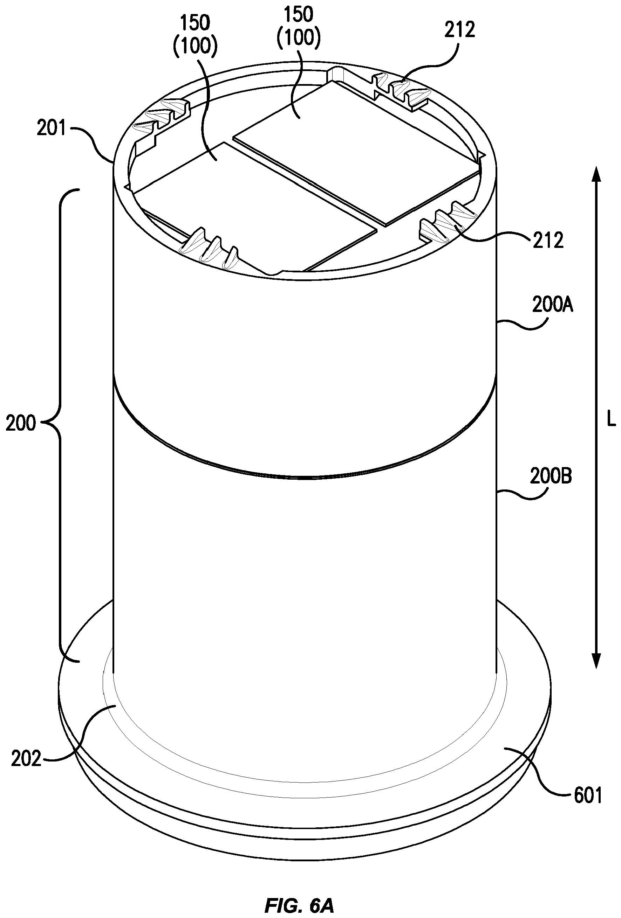

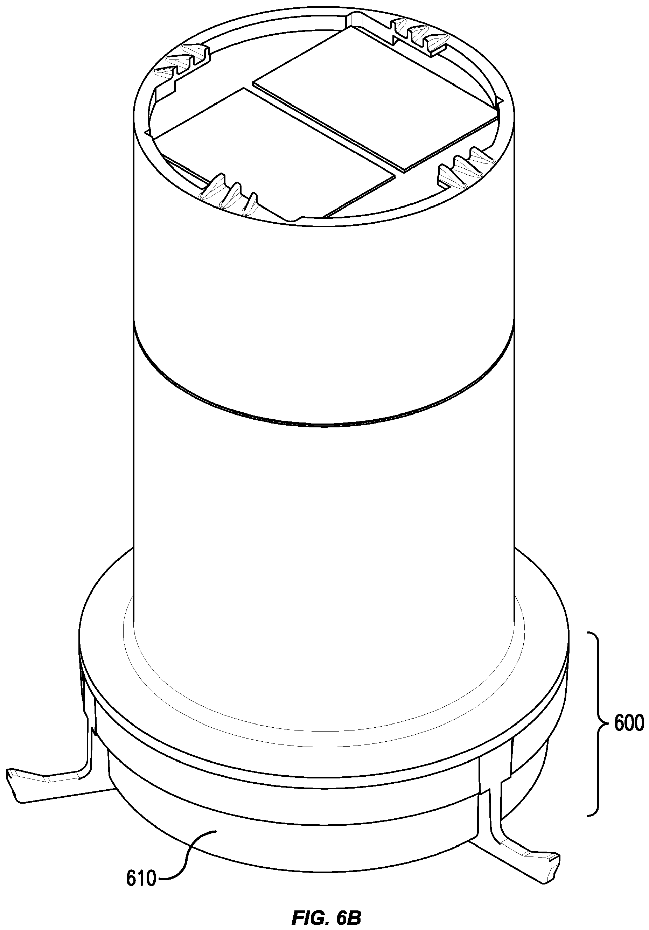

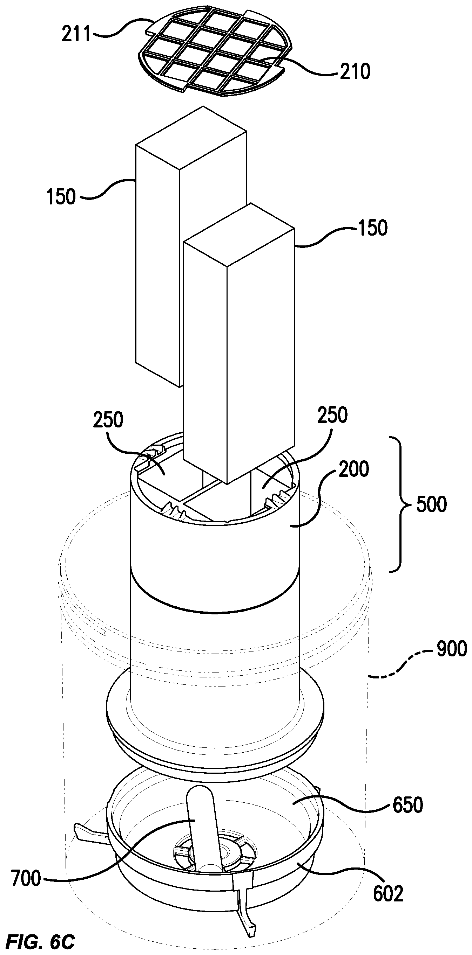

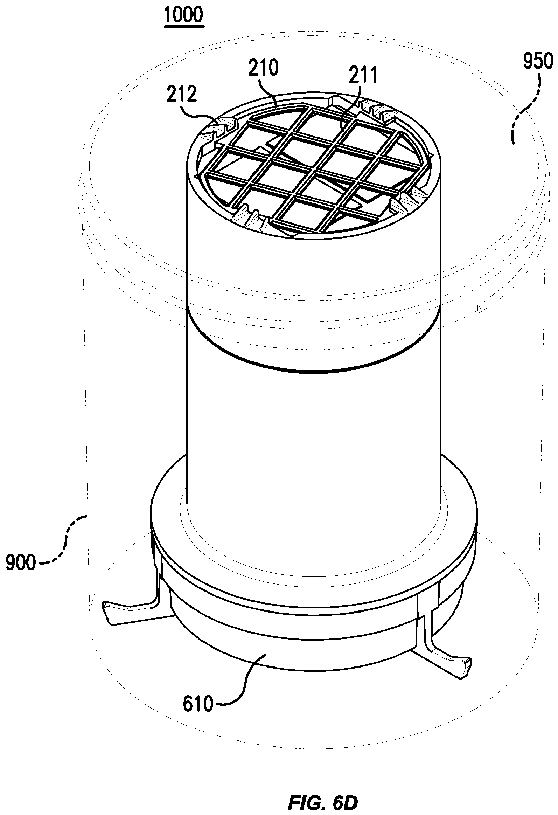

Figures 6A-6D are drawings showing a cell culture arrangement including two separate fixed beds, each comprising a pleated porous cell culture medium in a bed housing, wherein the cell culture arrangement is arranged in a vessel providing a cell culture device according to another aspect of the invention. Figure 6A shows an assembled cell culture arrangement, Figure 6B shows the assembled cell culture arrangement shown in Figure 6A and a pump arrangement; Figure 6C shows an exploded view of a cell culture device including an impeller arranged in the pump body below the bed housing; and Figure 6D shows a top isometric view of an assembled cell culture device, wherein in Figures 6C and 6D, the shipping cover and stabilizing element are removed so that a bioreactor lid can be placed on top before operating the device.

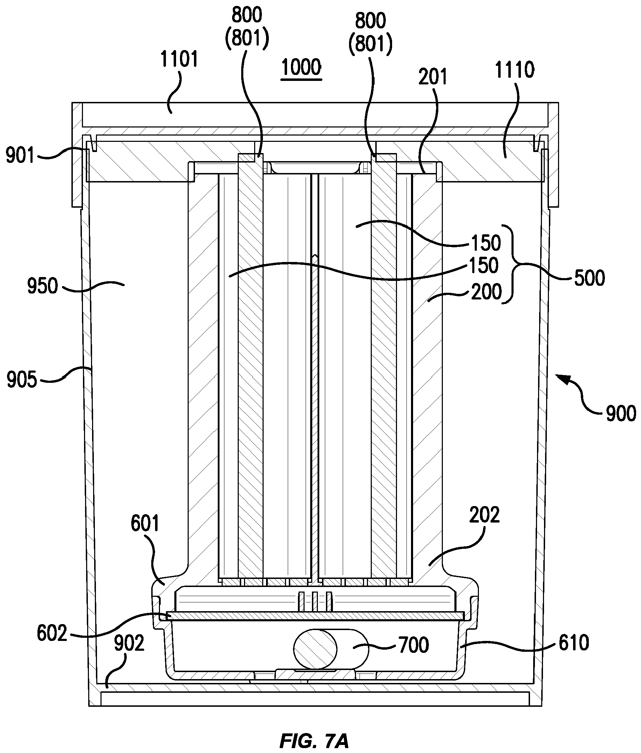

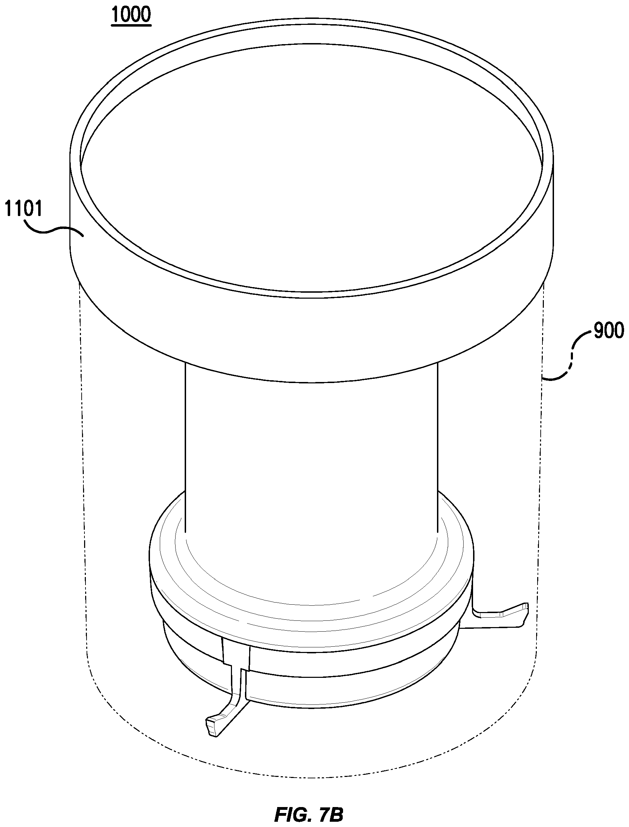

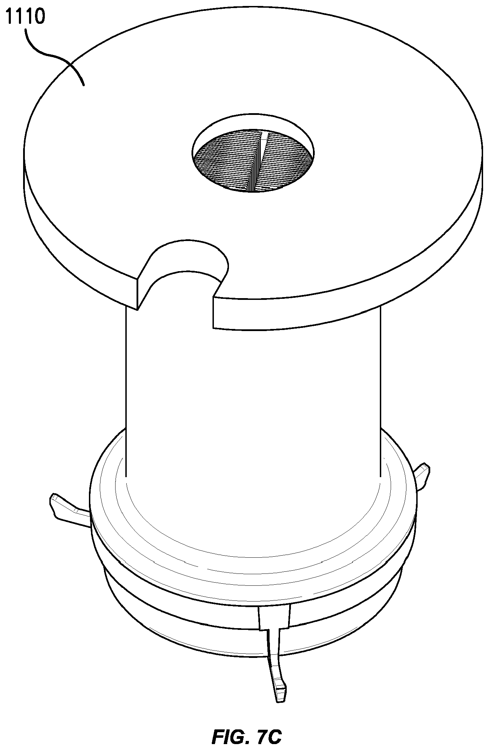

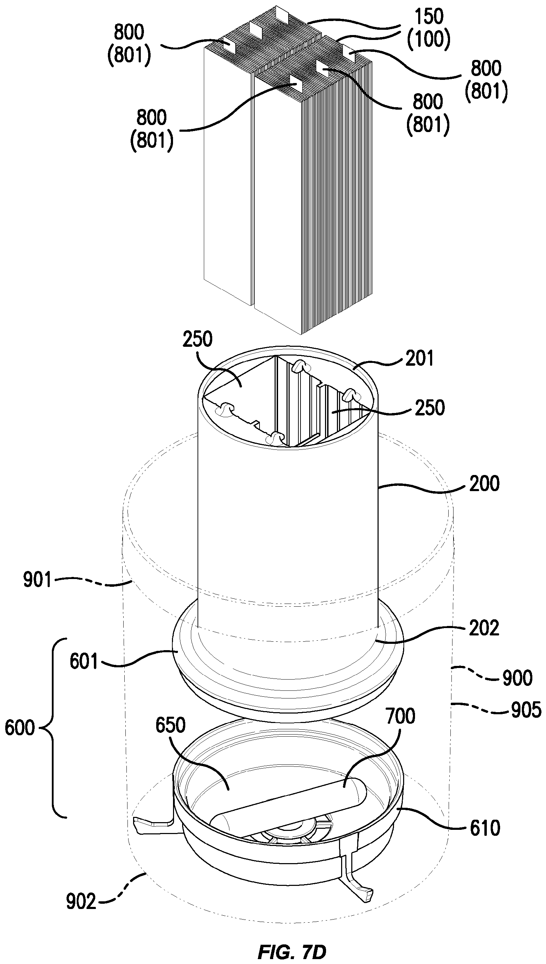

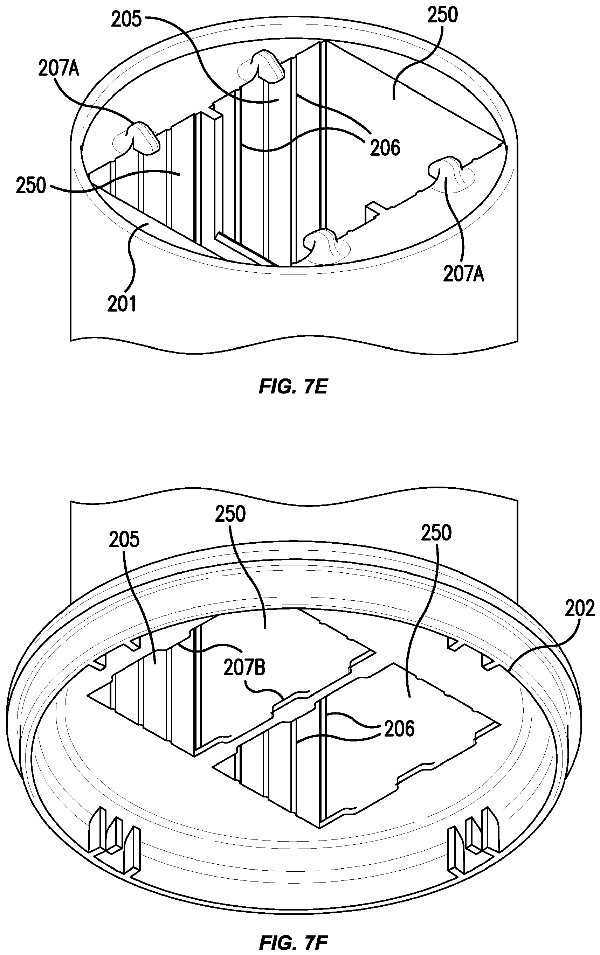

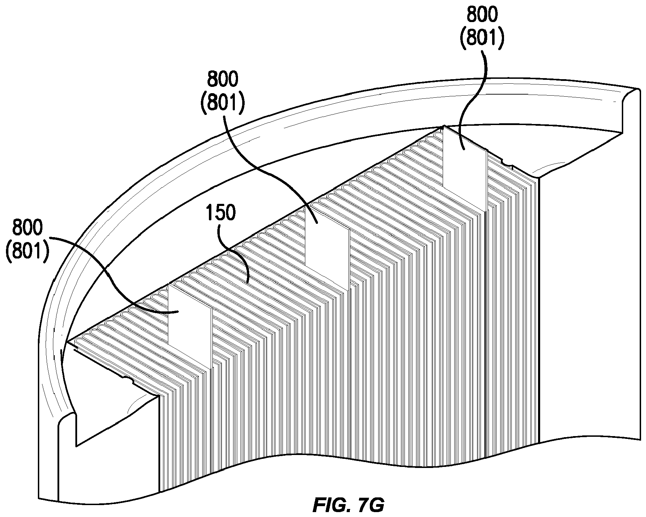

Figures 7A-7G are drawings showing a cell culture arrangement including two separate fixed beds, each comprising a pleated porous cell culture medium in a bed housing , wherein the cell culture arrangement is arranged in a vessel providing a cell culture device according to another aspect of the invention. Figure 7A shows a cross-sectional assembled view for shipping/storage, also showing the shipping cover and a fixed bed housing and a stabilizing element interposed between the shipping cover and the bed retainer, and Figure 7B shows a perspective assembled view of the cell culture device for shipping/storage, also showing a shipping cover. Figure 7C shows a perspective view of the stabilizing element after removal of the shipping cover. Figure 7D shows an exploded view of the cell culture device after removal of the stabilizing element, also showing removable sampling elements inserted in the pleated porous cell culture media of the fixed beds and an impeller arranged in the pump body below the bed housing. Figures 7E and 7F show, respectively, top and bottom views of the bed housing of the cell culture device, wherein the inner walls of the pump outer body include optional ribs to space the fixed beds away from the inner walls, and retainers to keep the fixed beds in the bed housing. Figure 7G shows a partial view of the cell culture device shown in Figure 7D, showing a plurality of removable sampling elements inserted in the pleated porous cell culture media of the fixed beds.

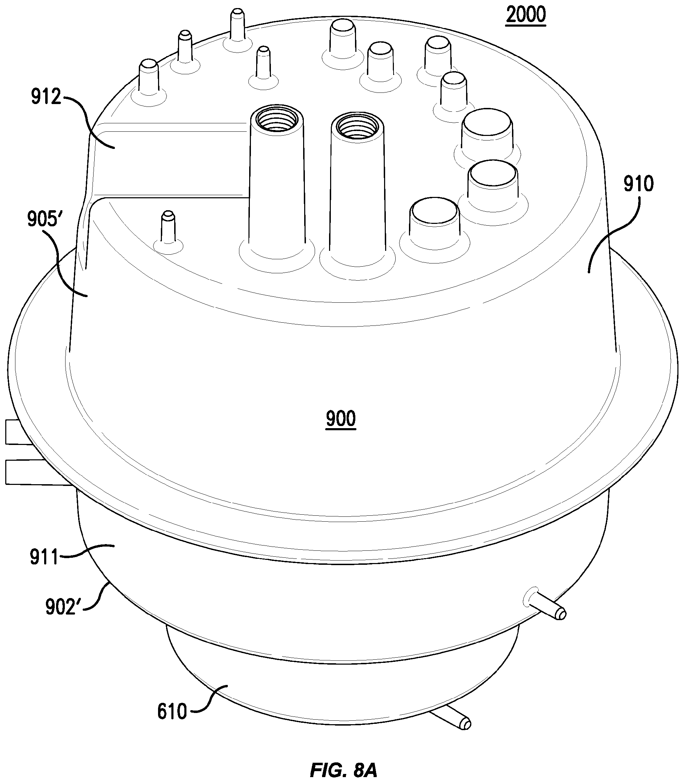

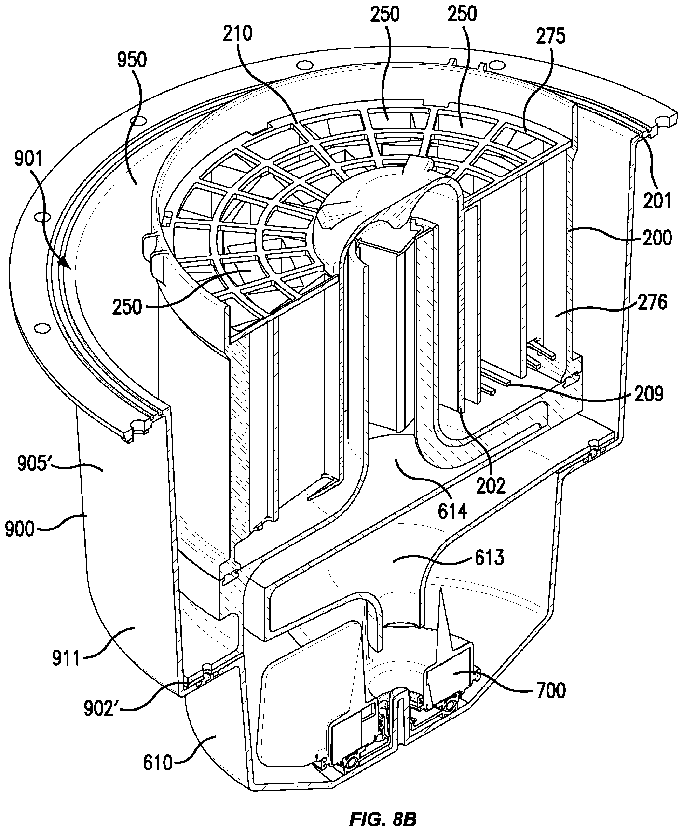

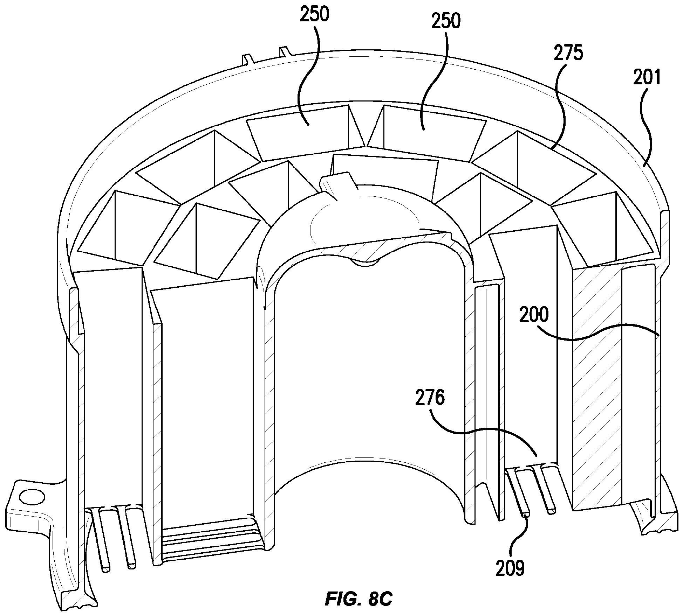

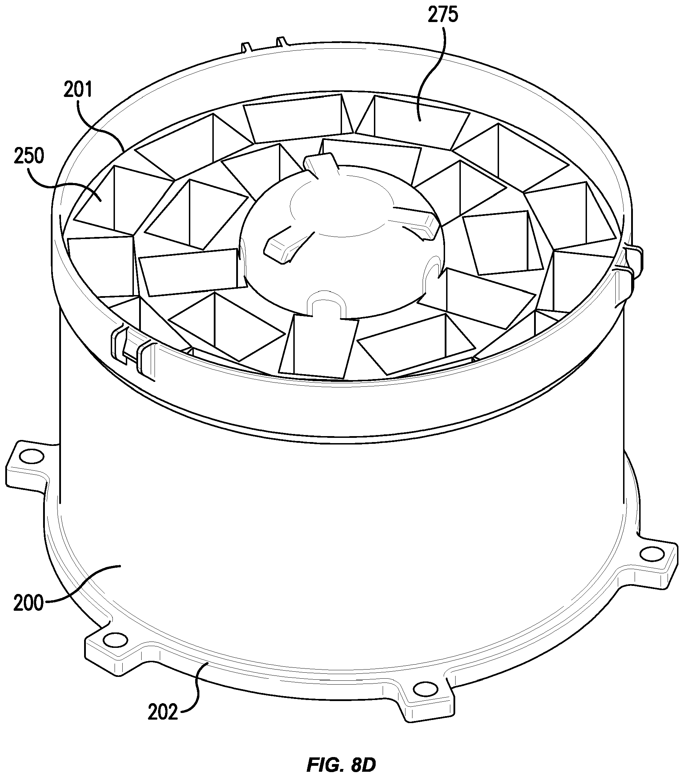

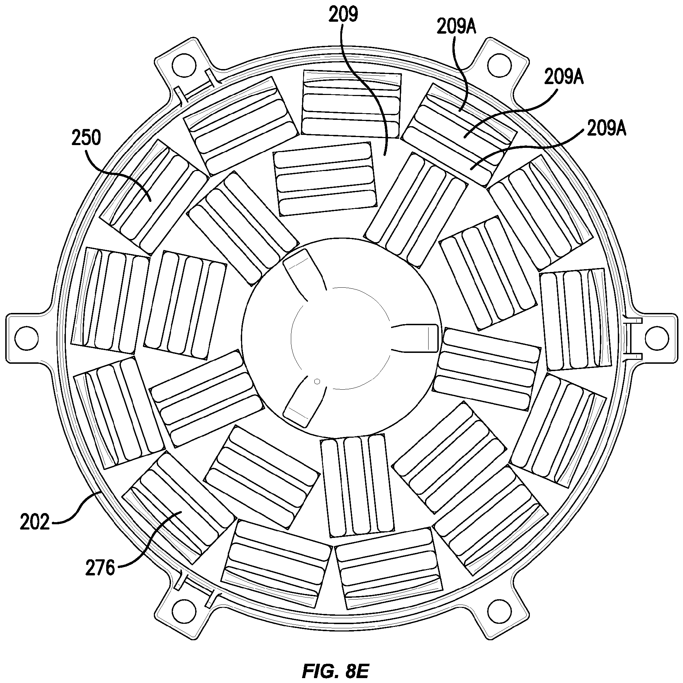

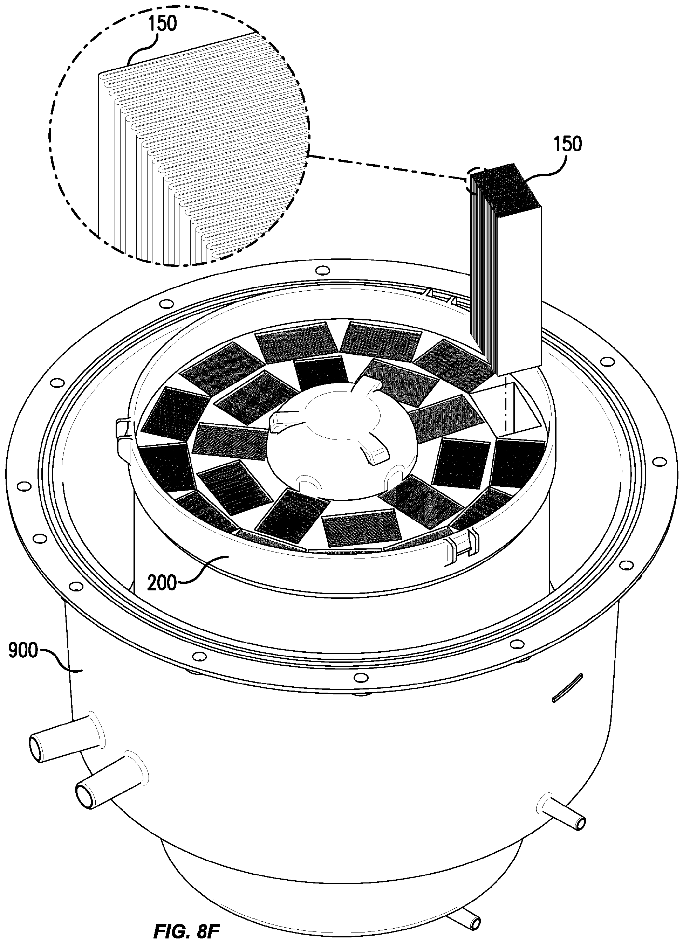

Figures 8A-8F are drawings showing a cell culture device comprising a cell culture arrangement including a plurality of separate fixed beds, each comprising a pleated porous cell culture medium, in a bed housing according to another aspect of the invention. Figure 8A shows a perspective view of an assembled cell culture device, also showing various optional ports, e.g., for sampling/determining various parameters during operation. Figure 8B shows a cross-sectional view of the cell culture device shown in Figure 8A with the upper housing section removed, also showing an upper baffle or grid plate, and an impeller arranged in the pump body below the bed housing as well as a central column S-bend and flow diverter in the middle of the fixed bed housing. Figure 8C shows a cross-sectional view of the bed housing with the upper grid plate removed; Figure 8D shows a top perspective view of the cell culture arrangement without the fixed beds in fixed bed chambers, Figure 8E shows the bottom of the bed housing, also showing a grid plate providing the bottom of 23 fixed bed chambers; and Figure 8F shows a top perspective view of the cell culture arrangement with the fixed beds in fixed bed chambers.

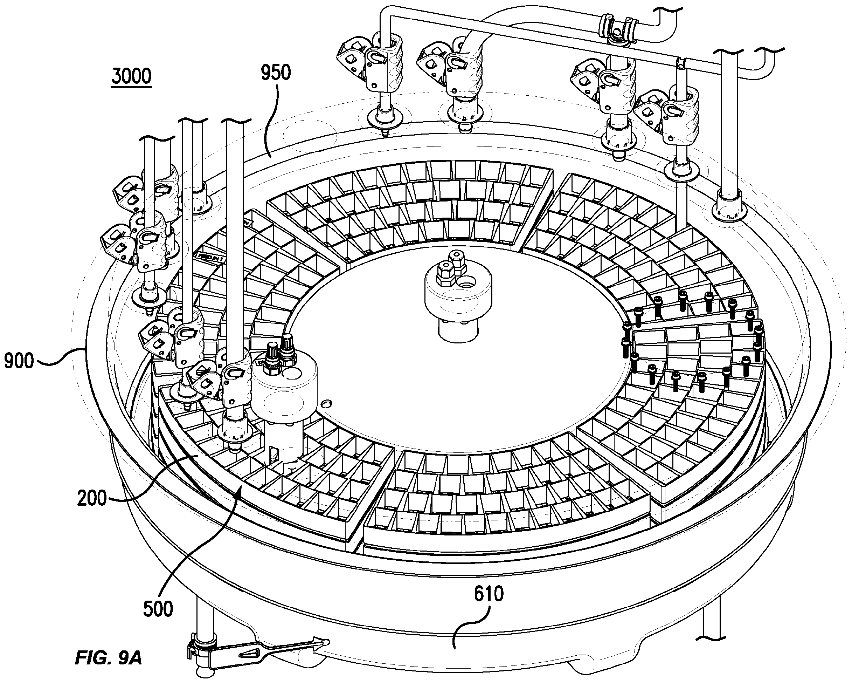

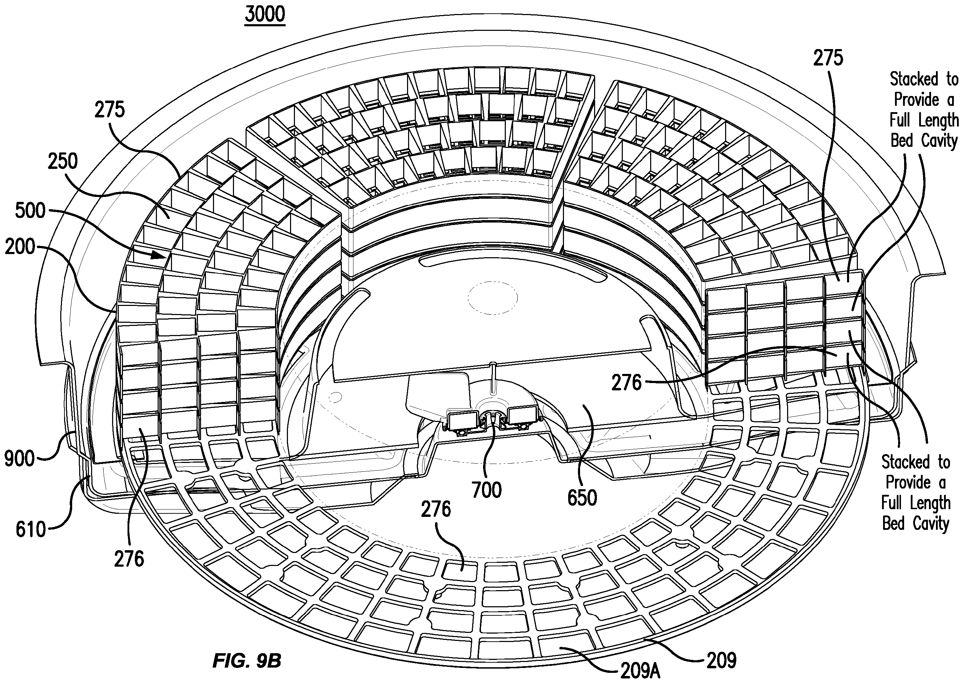

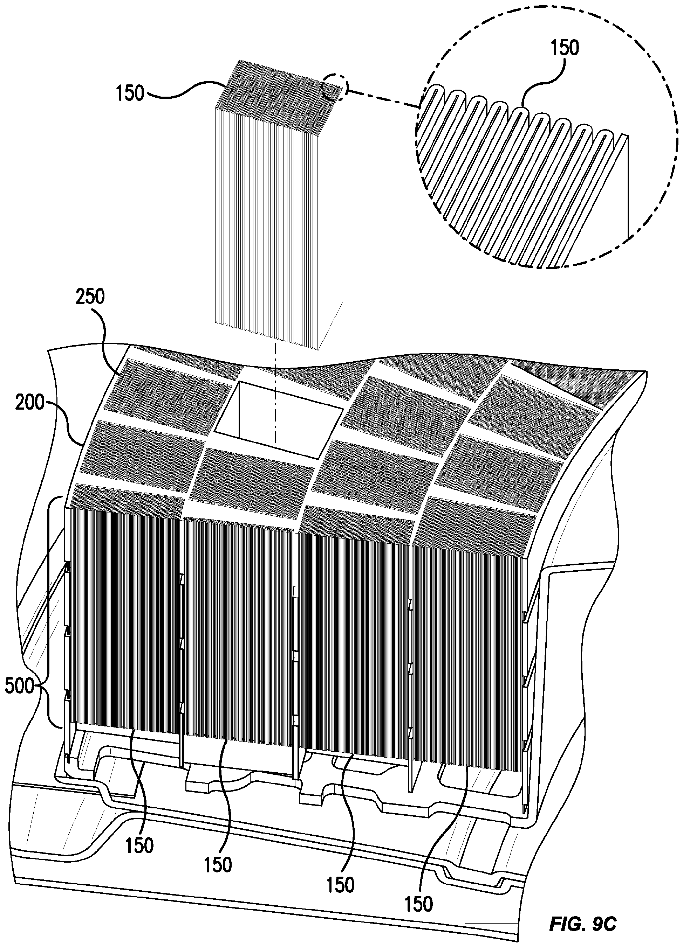

Figures 9A-9C are drawings showing a cell culture device comprising a cell culture arrangement including a plurality of stackable sections, the stacked sections providing vertically arranged fixed bed chambers receiving fixed beds (a single fixed bed received in a vertically arranged stacked fixed bed chambers providing a full length fixed bed cavity), each fixed bed comprising a pleated porous cell culture medium in a bed housing, according to another aspect of the invention. Figure 9A is a top perspective view (the cover is transparent), showing a cell culture arrangement including a plurality of stackable sections, providing vertically arranged fixed bed chambers to receive fixed beds, each fixed bed comprising a pleated porous cell culture medium; Figure 9B shows a partial cut-away view of the cell culture device shown in Figure 9A without the fixed beds in vertically arranged stacked fixed bed chambers, also showing the bottom of the culture arrangement, and an impeller arranged in the lower portion of the housing; and Figure 9C shows a partial cut-away view of the cell culture device shown in Figure 9A with fixed beds in vertically arranged stacked fixed bed chambers.

### DETAILED DESCRIPTION OF THE INVENTION

In accordance with an aspect of the invention, a cell culture arrangement is provided comprising (a) a housing having a first end and a second end and a longitudinal axis; and; (b) at least one fixed bed arranged in the housing, the at least one fixed bed comprising a continuous pleated porous medium comprising a top end, a bottom end, a front side, a rear side, a right side, and a left side, and a plurality of pleats folded on the right side and the left side, and having vertical fluid flow channels along the longitudinal axis between adjacent pleats.

In some aspects, the cell culture arrangement includes a plurality of removable sampling elements comprising porous media inserted in the continuous pleated porous medium.

In some aspects, the cell culture arrangement comprises at least two fixed beds arranged in the bed housing (each bed in a separate bed chamber 250), each of the at least two fixed beds comprising a continuous pleated porous medium comprising a top end, a bottom end, a front side, a rear side, a right side, and a left side, and a plurality of pleats folded on the right side and the left side, and having vertical fluid flow channels along the longitudinal axis between adjacent pleats.

In some aspects of the cell culture arrangement, the/each continuous pleated porous medium has a pleat density in the range of 25% to 95%, in some aspects, 40% to 95%, more typically, in the range of 40% to 85%. In some aspects, the cell culture arrangement includes at least two fixed beds arranged in the housing, each of the at least two fixed beds comprising a continuous pleated porous medium comprising a top end, a bottom end, a front side, a rear side, a right side, and a left side, and a plurality of pleats (each having first and second pleat legs) folded on the right side and the left side, and having vertical fluid flow channels along the longitudinal axis between adjacent pleats.

Typically, the pleated porous medium is oriented such that the pleats have a direction in a horizontal x-y-plane, and the vertical fluid flow channels are in a vertical z-plane.

In another aspect, a cell culture device comprises (a) an aspect of the cell culture arrangement; (b) a pump body arranged at the second end of the housing, the pump body including a central cavity; (c) an impeller arranged in the central cavity of the pump body; and; (d) a cell culture vessel having a central chamber, wherein the cell culture arrangement is arranged in the central chamber.

In another aspect, a cell culture device comprises (a) an aspect of the cell culture arrangement; (b) a pump assembly arranged at the second end of the housing, the pump assembly comprising a pump outer body and a pump body connected to the pump outer body, the pump body including a central cavity; (c) an impeller arranged in the central cavity of the pump body; and; (d) a cell culture vessel having an open first vessel end and a closed second vessel end, and a vessel side wall connected to the open first vessel end and the closed second vessel end, the cell culture vessel having a central chamber, wherein the cell culture arrangement and pump assembly are arranged in the central chamber.

In an aspect of the cell culture device, the pump assembly comprises a pump lid attached to the second end of the housing, and the pump outer body is connected to the pump lid. Alternatively, or additionally, in an aspect of the cell culture device, the impeller comprises a magnetic impeller.

Other aspects of the invention include culturing cells, processing cell culture liquids, and fixed-bed bioreactor systems.

A method of culturing cells according to an aspect of the invention comprises placing cell culture media and cells in an aspect of the cell culture device; and, operating the magnetic impeller, continuously supplying the fixed bed with aerated liquid culture medium containing nutrients and dissolved oxygen, and removing undesirable material resulting from biological processes, such as carbon dioxide and lactate. In some aspects, the method further comprises harvesting the cultured cells.

Advantageously, cell culture liquid (liquid culture media) flows between pleat legs, such that the pleats of the porous cell culture medium are "self-aligning" to provide, without spacers, desired suitable spacing between the pleat legs and adjoining pleats, while exhibiting low backpressure and shear.

Cell culture arrangements, cell culture devices, and cell culture systems can have a single fixed bed comprising a cell culture medium, or a plurality of fixed beds comprising cell culture media, e.g., two or more, 20 or more, 100 or more, or 240 or more, fixed beds.

Each of the components of the invention will now be described in more detail below, wherein like components have like reference numbers.

Using the aspects shown in Figures 1A-1D (components ending with "A," "a," and "a',") and 2A-2D (components ending with "B," "b," and "b',") for reference, a fixed bed 150A, 150B comprises a pleated porous cell culture medium 100A, 100B comprising a continuous pleated porous medium 50A, 50B, comprising a top end 51A, 51B, a bottom end 52A, 52B" a front side 53A, 53B, a rear side 54A, 54B, a right side 55A, 55B, and a left side 56A, 56B, and a plurality of pleats 60A, 60B, folded on the right side and the left side, and having vertical fluid flow channels 70A, 70B between the legs of the pleats.

Each pleat has a pair of pleat legs (61A, 62A; 61B, 62B), each pleat leg having a first pleat leg surface (61a, 62a; 61b, 62b) and a second pleat leg surface (61a', 62a'; 61b', 62b'), wherein portions of the first pleat leg surface and a second pleat leg surface in a pleat may contact each other, and portions of one first pleat leg surface may contact portions of the second pleat leg surface of an adjacent pleat, and during use, due to tangential flow, the vertical fluid flow channels are formed between pleat legs providing "self-spacing," (*see,* Figure 3A).

In some aspects, the fixed bed comprising the continuous pleated porous medium in a housing has a pleat density in the range of 25% to 95%, typically, in the range of 40% to 95%, preferably in the range of 40% to 85%. Typically, the pleated porous medium can be oriented such that the pleats have a direction in a horizontal x-y-plane, and the vertical fluid flow channels are in a vertical z-plane, wherein the closed edges of the pleats are parallel to vertical fluid flow.

Aspects of the fixed bed are scalable, and can have any suitable fixed bed height, e.g., in the range of 2 cm to 150 mm, typically in the range of 10 mm to 100 mm.

The fixed bed can have a variety of configurations, typically rectangular or square (as illustrated).

As shown in Figures 1A-1D and 2A-2D (showing "dry" media), pleats can have wider or narrower spacing ("a radius;" radius R1 in Figure 1C is greater than radius R2 in Figure 2C) at the inner surface of the pleats, the media providing flow channels having a closed end (formed by a pleat) and a open end (without a pleat), the flow channels alternating between closed at a first end and open at a second end, and open at the first end and closed at the second end). However, while it appears that portions of opposing surfaces contact each other, once the cell culture device is operated, during tangential flow, at least major portions of some the opposing surfaces will be spread apart, providing desired suitable spacing (*see,* Figures 1C-1D, 2C-2D, and 3A). Moreover, the liquid will penetrate multiple layers, portions of opposing surfaces remaining in contact will still function in cell culture.

In some aspects, the porous cell culture medium can be pleated using scrap material as "sacrificial layers" during the pleating process. For example, as shown in Figures 4A-4D, carrier media 171, 172 can be placed on either side of the porous cell culture medium and pleated together (e.g., using a corrugating machine), and the scrap material is subsequently removed as shown in Figure 4D. The resultant fixed bed can have a larger radius (e.g., "R1") as shown in Figure 1C, compared to fixed beds pleated without the use of sacrificial layers (e.g., having smaller radius "R2") as shown in Figure 2C.

In preferred aspects of the cell culture arrangement, wherein the fixed bed is arranged in a housing, the fixed bed pleat density is in the range of 40% to 85% to allow homogenous cell distribution in the bed due to cells traveling along the flow channels. Illustratively, using Figure 3B for reference, wherein fixed bed 150 has a fixed height of 100 mm and a pleat height of 40 mm, 66 pleats per 40 mm = 76% pleat density. 100% pleat density is when the combined thicknesses of all of the pleats inside the housing equals the width of the internal cavity of the housing (e.g., if the width of the internal cavity is 40 mm, and the thickness of each leg of the pleat is .23 mm (making the thickness of the pleat .46 mm), 40 mm/.46 mm=87 pleats for 100% pleat density). In this configuration, the entire housing is filled with carrier material and no space is available in between. To allow for flow channels, the number of pleats is reduced to less than 100% of the theoretical maximal amount of pleats that fit in the housing.

The continuous pleated porous medium can have any number of pleats. For example, the fixed bed can have in the range of 50 to 60 pleats, but can less than 50 pleats or more than 60 pleats.

In accordance with aspects of the invention, the porous cell culture medium, which is flexible, can be a woven or a non-woven porous cell culture medium or a porous membrane, and can be formed from any of numerous materials, including those known in the art, including, for example, a natural or, more preferably, a synthetic polymer, such as polyethylene terephthalate (PET). Suitable porous cell culture media are commercially available. One non-woven porous cell culture medium is non-woven hydrophilized PET. The porous cell culture medium typically has a thickness in the range of 50 to 400 µm.

The porous cell culture medium can have any suitable pore structure, e.g., a pore size (for example, as evidenced by bubble point, or by K_{L} as described in, for example, U.S. Patent 4,340,479, or evidenced by capillary condensation flow porometry), a mean flow pore (MFP) size (e.g., when characterized using a porometer, for example, a Porvair Porometer (Porvair plc, Norfolk, UK), or a porometer available under the trademark POROLUX (Porometer.com; Belgium)), a pore rating, a pore diameter (e.g., when characterized using the modified OSU F2 test as described in, for example, U.S. Patent 4,925,572), or removal rating media.

The porous cell culture medium can have any desired critical wetting surface tension (CWST, as defined in, for example, U.S. Patent 4,925,572). The CWST can be selected as is known in the art, e.g., as additionally disclosed in, for example, U.S. Patents 5,152,905, 5,443,743, 5,472,621, and 6,074,869. The surface characteristics of the porous cell culture medium can be modified (e.g., to affect cell attachment, to affect the CWST, to include a surface charge, e.g., a positive or negative charge, and/or to alter the polarity or hydrophilicity of the surface) by wet or dry oxidation, by coating or depositing a polymer or hydrogel on the surface, or by a grafting reaction. Modifications include, e.g., irradiation, a polar or charged monomer, coating and/or curing the surface with a charged polymer, and carrying out chemical modification to attach functional groups on the surface. Grafting reactions may be activated by exposure to an energy source such as gas plasma, vapor plasma, corona discharge, heat, a Van de Graff generator, ultraviolet light, electron beam, or to various other forms of radiation, or by surface etching or deposition using a plasma treatment.

In some aspects (*see,* for example, Figures 7A, 7D and 7G), the cell culture arrangement includes a plurality of removable sampling elements 800 comprising porous media 801 inserted in the continuous pleated porous medium (e.g., inserted in the flow channels between pleat legs). If desired, the sampling elements comprise single sheets of porous media, wherein the porous media is identical to the porous media using for producing the continuous pleated porous medium. If desired, the removable sampling elements can include pull tabs attached to the sampling elements, or the length of the sampling element extending beyond (sticking out beyond) the continuous pleated porous medium provides the pull tab.

Sampling elements can be used to, for example, determine the cell population density of the cells adhered to the sampling element. Alternatively, or additionally, analyzing the sample comprises one or more of replication competent retroviral testing, adventitious virus testing, and sterility testing (e.g., wherein the sampling element with adhered cells is placed in liquid media to analyze for exogenous microbial growth).

In accordance with aspects of the invention, a cell culture arrangement is provided comprising (a) a housing having a first end and a second end and a longitudinal axis; and; (b) at least one fixed bed arranged in the housing, the at least one fixed bed comprising (*see, for example,* Figures 1A-1D and 2A-2D) a continuous pleated porous medium comprising a top end, a bottom end, a front side, a rear side, a right side, and a left side, and a plurality of pleats folded on the right side and the left side, the pleats having legs, and having vertical fluid flow channels along the longitudinal axis between adjacent pleats.

As shown in Figures 5A-5D, 6A-6D, 7A-7G, 8A-8F, and 9A-9C, a fixed bed 150 comprising a pleated porous cell culture medium 100 is arranged in a bed housing 200 having a first end 201, a second end 202, a bed chamber 250, and a longitudinal axis L, providing a cell culture arrangement 500 (Figures 6A-6D and 7A-7G show two fixed beds 150 each comprising a pleated porous cell culture medium 100 arranged separate bed chambers 250 in the same bed housing 200; Figures 8A-8F, and 9A-9C show more than two fixed beds 150 each comprising a pleated porous cell culture medium 100 arranged in separate bed chambers 250 in the same bed housing 200). The bed housing can be a single piece from the first end to the second end (as shown in Figures 7A and 8B), or can comprise multiple segments fitted together. For example, the illustrated housings shown in Figures 5D, and 6A-6D have an upper segment 200A fluid tightly engaged with a lower segment 200B, and a similar result is provided with the vertically arranged stacked fixed bed chambers shown in Figures 9A-9C (stackability provided for ease of manufacturing).

In some aspects, as shown in more detail in Figures 6C, 6D, and 8B, a bed retainer (such as a baffle or grid plate) 210 having apertures 211 is arranged at the first end 201 of the housing, and, in some aspects can be engaged with the housing, e.g., by rotation such that portions of the retainer fit in slots 212 at the first end 201 as shown in Figure 6D.

In the aspects shown in Figures 7E and 7F, the bed housing 200 has inner walls 205 including optional outwardly extending ribs 206 spacing the cell culture medium 100 away from the walls, the first end 201 of the bed housing including optional inwardly facing projections 207A and the second end 202 of the bed housing including optional inwardly facing projections 207B retaining the cell culture medium in the bed housing 200.

In the aspects as shown in Figures 5A-5D, 6C-6D, 7A, and 7D, a cell culture device 1000 includes the cell culture arrangement 500; a pump assembly 600 arranged at the second end 202 of the bed housing 200, the pump assembly comprising an optional pump lid 601 attached to the second end of the housing, a pump outer body 602 connected to the pump lid, and a pump body 610 connected to the pump outer body 602, the pump body 610 including a central cavity 650; a magnetic drive impeller 700 arranged in the central cavity 650 of the pump body 610; and; a cell culture vessel 900 having an open first vessel end 901 and a closed second vessel end 902, and a vessel side wall 905 connected to the open first vessel end and the closed second vessel end, the cell culture vessel having a central chamber 950, wherein the cell culture arrangement and pump assembly are arranged in the central chamber.

Aspects of cell culture devices can include any number of ports, and can include at least one inlet port and at least one outlet port. For example, Figure 8A shows a perspective view of an assembled cell culture device, also showing various optional ports at the upper end of the device, e.g., for sampling/determining various parameters during operation, as well as an inlet port and an outlet port. Using the aspect shown in Figure 8A for reference, in another aspect, the upper end of the device (upper surface of first cell culture housing 910) includes a large port, e.g., for receiving a pump assembly and/or a depression 912 for receiving another component.

The illustrated aspects of the cell culture device shown in Figure 5D and 7A has a closed shipping/storage cover 1101 fluid tightly covering the open first vessel end 901, wherein an optional stabilizing element 1110 (*see also,* Figure 7C) such as a blocking foam is arranged between the inside surface of the closed cover 1101 and the bed retainer 210. Before using the cell culture device, the lid 1101 and stabilizing element 1110 (if present) are removed (*see,* Figures 6C-6D and 7A), and a bioreactor lid (e.g., a stainless steel cover) is fluid tightly placed on top of the bed retainer.

Typically, the cell culture device has at least one structure (e.g., via one or more connectors on a bioreactor system lid or integrated into the device) allowing a transfer of cell culture medium (liquid) essentially free of cells.

As noted above, a cell culture arrangement and/or a cell culture device can have a single fixed bed or a plurality of fixed beds each comprising a pleated porous cell culture medium.

For example, using Figures 6C and 7D for reference, cell culture device 1000 includes cell culture arrangement 500 including a first fixed bed 150 and a second fixed bed 150 arranged in separate bed chambers 250 in the bed housing 200. In other aspects, a cell culture device includes more than two fixed beds, for example, the aspect of the cell culture device 2000 shown in Figure 8F has over 20 fixed beds in separate bed chambers 250 in the bed housing 200, and the aspect of the cell culture device 3000 shown in Figures 9A-9C has over 200 fixed beds in separate stacks of bed chambers 250 in the bed housing 200.

Using Figures 8A-8F for reference, the illustrated aspect of cell culture device 2000 includes cell culture arrangement 500 including more than two fixed beds 150, for example, 10 or more fixed beds (23 fixed beds 150 in separate bed chambers 250 are illustrated, *see, for example,* Figure 8F) arranged in the bed housing 200. Each bed chamber 250 includes a first chamber end 275 and a second chamber end 276, wherein the bed housing has a grid plate 209 at the second end of the bed housing. If desired, bed spacers (that allow fluid to pass along the spacers to the beds) can be arranged in any aspect of the cell culture arrangement, bed housing and/or each bed chamber according to the invention, e.g., with the spacers arranged below the beds 150, wherein the spacers are retained in the device by a grid plate 209 at the second chamber end/second bed housing end. The grid plate has at least one slot 209A (3 are illustrated) allowing fluid flow arranged at each second chamber end. In some aspects, the use of spacers is desirable so that the same bed housing can be used to accommodate fixed beds of different lengths.

In the aspect of the cell culture device 2000 shown in Figures 8A-8F, the device includes a pump body 610 having central cavity 650 arranged below the second end 202 of the bed housing 200, with a magnetic drive impeller 700 arranged in the central cavity 650 of the pump body 610, with a flow diverter 613 that acts both as an outlet toward the central column 614 with an S-bend above the impeller leading to the central column, providing an inlet for the liquid at the center of the impeller coming from the outer chamber 950 after passing through the fixed beds and coming down toward the impeller, wherein the bed housing is arranged in a cell culture vessel 900 having a first (upper) cell culture housing 910, and a second (lower) cell culture housing 911, the second cell culture housing having an open first vessel end 901 and an open second vessel end 902' and a vessel side wall 905' connected to the open first vessel end and the second vessel end, the cell culture vessel having a central chamber 950, wherein the cell culture arrangement is arranged in the central chamber.

Using Figures 9A-9C for reference, the illustrated aspect of cell culture device 3000 includes cell culture arrangement 500 including more than two fixed beds 150, for example, over 200 fixed beds 150 in separate bed chambers 250 are illustrated arranged in the bed housing 200. Each bed chamber 250 includes a first chamber end 275 and a second chamber end 276 and a grid plate 209 at the second chamber end/second bed housing end. The grid plate has at least one slot 209 allowing fluid flow arranged at each second chamber end. In the illustrated aspect of cell culture device 3000 the cell culture arrangement includes a plurality of stackable sections, the stacked sections providing vertically arranged fixed bed chambers receiving fixed beds (a single fixed bed received in a vertically arranged stacked fixed bed chambers providing a full length fixed bed cavity as shown in Figure 9B), each fixed bed comprising a pleated porous cell culture medium in a bed housing, according to another aspect of the invention.

In the aspect of the cell culture device 3000 shown in Figures 9A-9C, the device includes a pump body 610 having central cavity 650 arranged below the second end 202 of the bed housing 200, with a magnetic drive impeller 700 arranged in the central cavity 650 of the pump body 610, wherein the bed housing is arranged in a cell culture vessel 900 having an open first vessel end 901 and an open second vessel end 902' and a vessel side wall 905' connected to the open first vessel end and the second vessel end, the cell culture vessel having a central chamber 950, wherein the cell culture arrangement is arranged in the central chamber.

Typically bioreactor systems according to aspects of the invention can be operated as known in the art (*see,* for example, the iCELLis^{®} Single-Use Fixed-Bed Bioreactor System, including the iCELLis^{®} Nano System and the iCELLis^{®} 500 System (Pall Corporation, Port Washington, NY, USA)), and can include at least one, preferably, at least two, sensors such as sensors for dissolved oxygen, pH, temperature, e.g., as used in commercially available fixed-bed bioreactor systems, for example, the iCELLis^{®} Single-Use Fixed-Bed Bioreactor System, including the iCELLis^{®} Nano System and the iCELLis^{®} 500 System (Pall Corporation, Port Washington, NY, USA).

The bed housing can be fabricated from any suitable impervious material including any rigid impervious thermoplastic material, which is compatible with the fluid being processed. For example, the housing can be fabricated from a metal, such as stainless steel, or from a polymer. In some aspects, the housing is a polymer such as a plastic, in some aspects, an acrylic, polypropylene, polystyrene, polyethylene, polyethylene terephthalate, or a polycarbonated resin.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred aspects of this invention are described herein, including the best mode known to the inventors for carrying out the invention. Variations of those preferred aspects may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A cell culture arrangement comprising
(a) a housing having a first end and a second end and a longitudinal axis; and;
(b) at least one fixed bed arranged in the housing, the at least one fixed bed comprising a continuous pleated porous medium comprising a top end, a bottom end, a front side, a rear side, a right side, and a left side, and a plurality of pleats folded on the right side and the left side, and having vertical fluid flow channels along the longitudinal axis between adjacent pleats.

2. The cell culture arrangement of claim 1, wherein the continuous pleated porous medium has a pleat density in the range of 25% to 95%.

3. The cell culture arrangement of claim 1 or 2, including at least two fixed beds arranged in the housing, each of the at least two fixed beds comprising a continuous pleated porous medium comprising a top end, a bottom end, a front side, a rear side, a right side, and a left side, and a plurality of pleats folded on the right side and the left side, and having vertical fluid flow channels along the longitudinal axis between adjacent pleats.

4. The cell culture arrangement of any one of claims 1-3, including a plurality of removable sampling elements comprising porous media inserted in the continuous pleated porous medium.

5. A cell culture device comprising:
(a) the cell culture arrangement according to any one of claims 1-4;
(b) a pump body arranged at the second end of the bed housing, the pump body including a central cavity;
(c) an impeller arranged in the central cavity of the pump body; and;
(d) a cell culture vessel having a central chamber, wherein the cell culture arrangement is arranged in the central chamber.

6. A method of culturing cells, the method comprising:
placing cell culture media and cells in the cell culture device of claim 5; and, operating the impeller.

7. The method of claim 6, further comprising harvesting the cultured cells.
